# EUROPEAN PATENT SPECIFICATION

(11) **EP 4 551 164 B1**
(45) Date of publication and mention of the grant of the patent: **26.08.2026**
(21) Application number: 23748098.3
(22) Date of filing: 30.06.2023
(51) Int. Cl.: A61F 2/44

(54) **INTERVERTEBRAL DEVICES**
ZWISCHENWIRBELVORRICHTUNGEN
DISPOSITIFS INTERVERTÉBRAUX

(30) Priority: 06.07.2022 GB 202209948
(43) Date of publication of application: 14.05.2025
(73) Proprietor: Axis Spine Technologies Ltd, Leeds LS15 8ZB (GB)
(72) Inventor: ARCOS, Jonathan, Reading Berkshire RG1 3EU (GB); HEUER, Frank, Reading Berkshire RG1 3EU (GB)
(74) Representative: Peter, Kenneth William
(86) International application number: PCT/GB2023/051729
(87) International publication number: WO 2024/009066

(56) References cited:
- WO-A1-2013/148176
- WO-A1-2021/058971
- WO-A1-2021/148794
- WO-A1-98/48739
- WO-A2-03/032812
- US-A- 5 653 763
- US-A1- 2013 103 153
- US-A1- 2023 240 857

## Description

### Field of the Invention

The present invention relates to intervertebral fusion devices and more specifically to modular intervertebral fusion devices.

### Background Art

Adjacent vertebrae in the spinal column are coupled to each other by a number of ligaments and the intervertebral disc. These anatomic structures hold the adjacent vertebrae together while allowing motion. Among these structures, the intervertebral disc functions as a cushion between the vertebrae whilst allowing for relative movement of the vertebrae. Problems with intervertebral discs arise from one or more of a range of diseases and conditions. A surgical procedure, such as spinal fusion, may be used to address such problems. The goals of spinal fusion include decompressing surrounding neural structures, re-establishing anatomic spinal alignment and stabilising the motion segment by having one vertebral body fuse, or heal, to the adjacent vertebral body. A typical spinal fusion procedure involves partial or full removal of a problematic intervertebral disc and installation of an intervertebral device in the place of the partially or fully removed intervertebral disc in order to maintain the intervertebral space height and alignment and facilitate the fusion of one vertebra to the next.

A known form of intervertebral device is the modular intervertebral device which comprises superior and inferior plates and a core component. The superior and inferior plates and a core component are separate components. The core component is sized and shaped to determine a separation between the superior and inferior plates. When the superior and inferior plates face each other, the core component is inserted between the superior and inferior plates to bring adjacent ones of the superior and inferior plates and the core component into engagement.

A spinal fusion procedure may be carried out by way of one of several different techniques. Posterior lumbar interbody fusion (PLIF) is one such technique in which the patient's spine is approached from an incision in the middle of the back (WO03/032812 A2 discloses an example thereof). Anterior lumbar interbody fusion (ALIF) is another such technique in which the patient's spine is approached from the opposite direction, i.e. from an incision on the anterior side of the patient. A third such technique is lateral lumbar interbody fusion (LLIF; WO2021/058971 A1 discloses an example thereof) in which the patient's spine is approached from an incision at the side of the patient's waist. The incision is usually small. When the incision has been made the surgeon uses dilation tubes to form a tunnel which extends from the incision to the spine with the abdominal organs on one side of the tunnel and the spine muscles on the other side of the tunnel. LLIF minimises cutting of the spine muscles, minimises disturbance to the abdominal organs, and involves use of a single port to access the intervertebral space whereby LLIF can be performed in a minimally invasive fashion.

The present inventors have become appreciative of the advantage of minimising the profile of a lateral lumbar interbody fusion (LLIF) device whereby the extent of the incision and of the tunnel may be minimised. The present invention has been devised in light of the inventors' appreciation. It is therefore an object for the present invention to provide an improved lateral lumbar interbody fusion (LLIF) device and more specifically an improved modular LLIF device.

### Statement of Invention

The present invention relates to a device as claimed hereafter. Preferred embodiments of the invention are set forth in the dependent claims. While the implantation methods described herein do not form part of the invention, they are disclosed as they represent useful background for understanding the invention.

According to a first aspect of the present invention there is provided a modular lateral lumbar interbody fusion (LLIF) device comprising:
a superior component having a superior component top side and a superior component bottom side, the superior component configured to be received in an intervertebral space between first and second vertebrae whereby the superior component top side abuts against the first vertebra;
an inferior component having an inferior component top side and an inferior component bottom side, the inferior component configured to be received in the intervertebral space between the first and second vertebrae whereby the inferior component bottom side abuts against the second vertebra, the superior component bottom side and the inferior component top side opposing each other when the superior and inferior components are received in the intervertebral space; and
a core component configured for insertion between the superior and inferior components whereby a separation between the superior and inferior components and hence height of the LLIF device are determined,
wherein first and second superior rails protrude from the superior component bottom side, the first and second superior rails extending in the lateral direction, the first and second superior rails substantially parallel to each other and spaced apart from each other in a direction substantially orthogonal to the lateral direction,
wherein first and second inferior rails protrude from the inferior component top side, the first and second inferior rails extending in the lateral direction, the first and second inferior rails substantially parallel to each other and spaced apart from each other in a direction substantially orthogonal to the lateral direction,
wherein the first and second superior rails and the first and second inferior rails are disposed on their respective components such that the first and second superior rails interdigitate with the first and second inferior rails when the superior and inferior components are in registration and abut against each other before insertion of the core component between the superior and inferior components, and
wherein the core component is configured to inter-engage with each of the first and second superior rails and each of the first and second inferior rails during insertion of the core component between the superior and inferior components.

The modular lateral lumbar interbody fusion (LLIF) device comprises three main components, namely a superior component, an inferior component and a core component. The LLIF device is modular. In other words, the superior and inferior components and the core component are separate components. The superior and inferior components are therefore not attached to each other before assembly of the LLIF device. The superior and inferior components may be attached to each other solely by the core component when the LLIF device has been assembled.

In use, the superior and inferior components are placed in an intervertebral space between first and second vertebrae formed by at least partial removal of a problematic intervertebral disc. The superior component has a superior component top side and a superior component bottom side with the superior component placed in the intervertebral space such that the superior component top side faces the first vertebra or what might remain of a partially removed intervertebral disc. The inferior component has an inferior component top side and an inferior component bottom side with the inferior component placed in the intervertebral space such that the inferior component bottom side faces the second vertebra or what might remain of a partially removed intervertebral disc. The superior component bottom side and the inferior component top side oppose each other when the superior and inferior components are received in the intervertebral space. The superior and inferior components may be in registration with each other when in the intervertebral space and more specifically when the core component is fully inserted between the superior and inferior components, as described below. Introduction of the superior and inferior components without the core component into the intervertebral space allows for them to be moved around the adjacent nerve structures and in particular the nerve roots of the spinal cord, and with reduced requirement for retraction of the adjacent nerve structures. Risk of damage to the adjacent nerve structures is therefore reduced.

The core component is configured for insertion between the superior and inferior components. Upon insertion the core component determines a separation between the superior and inferior components and hence a height of the LLIF device with the superior component top side abutting against the first vertebra or what remains of the partially removed intervertebral disc and with the inferior component bottom side abutting against the second vertebra or what remains of the partially removed intervertebral disc. Differing heights of LLIF device may be provided by selection from plural core components of different height.

The superior component bottom side and the inferior component top side, as discussed below, determines the direction of insertion such that it is in the lateral direction, i.e. from the side of the waist towards the spine of the patient. Furthermore, the LLIF device is raised to full height by insertion of the core component when the superior and inferior components are present in the intervertebral space thereby presenting less risk of damage than insertion of an already full height LLIF device into the intervertebral space. In addition, assembly of the LLIF device in-situ improves scope for deformity correction through selection from core components that may provide for different correction angles. Provision for different correction angles may be inessential whereby the superior component top side and the inferior component bottom side are not inclined to each other when the LLIF device has been assembled.

Alternatively or in addition, at least one of the superior and inferior components may be configured to provide a correction angle. More specifically, the top and bottom sides of the respective component may be inclined to each other whereby the component is wedge shaped.

First and second superior rails protrude from the superior component bottom side. The first and second superior rails extend in the lateral direction and are substantially parallel to each other. The first and second superior rails are spaced apart from each other in a direction substantially orthogonal to the lateral direction. The first and second superior rails may be straight.

First and second inferior rails protrude from the inferior component top side. The first and second inferior rails extend in the lateral direction and are substantially parallel to each other. The first and second inferior rails are spaced apart from each other in a direction substantially orthogonal to the lateral direction. The first and second inferior rails may be straight.

The first and second superior rails and the first and second inferior rails are disposed on their respective components such that the first and second superior rails interdigitate with the first and second inferior rails when the superior and inferior components are in registration and abut against each other. The core component is configured to inter-engage with each of the first and second superior rails and each of the first and second inferior rails during insertion of the core component between the superior and inferior components.

The first and second superior rails and the first and second inferior rails may provide structure for inter-engagement with the core component during insertion of the core component between the superior and inferior components. The first and second superior rails and the first and second inferior rails may further provide structure for inter-engagement with the core component when the LLIF device has been assembled, i.e. when the core component has been fully inserted between the superior and inferior components whereby the core component is in registration with the superior and inferior components. Further to this, disposition of the first and second superior rails and the first and second inferior rails such that the first and second superior rails interdigitate with the first and second inferior rails when the superior and inferior components are in registration and abut against each other means the superior and inferior components may occupy less space when they are introduced into the intervertebral space and before insertion of the core component. This means a smaller incision and a smaller tunnel may be needed. Furthermore, the smaller space occupied by the superior and inferior components allows for their ease of placement in the intervertebral space ahead of insertion of the core component. The superior and inferior components may thus have structure for inter-engagement with the core component while the disposition of the rails may enable the superior and inferior components to occupy less space.

As mentioned above, the first and second superior rails interdigitate with the first and second inferior rails when the superior and inferior components are in registration. When the superior and inferior components are in registration the superior component bottom side and the inferior component top side may be substantially coextensive. The LLIF device may be configured such that the superior and inferior components are in registration during insertion of the core component. Furthermore, the LLIF device may be configured such that the superior and inferior components are in registration when the core component has been fully inserted between the superior and inferior components. The core component may be sized such that the core component extends in the lateral direction and the sagittal direction (i.e. in a direction orthogonal to the lateral direction) no further than the extent of the superior and inferior components when the LLIF device has been assembled.

The first and second superior rails may be non-equidistantly spaced from a laterally extending plane which bisects the superior component, and the first and second inferior rails may be non-equidistantly spaced from a laterally extending plane which bisects the inferior component. Furthermore, the first superior rail may be spaced apart from the laterally extending plane by a first rail distance further than the second superior rail is spaced apart from the laterally extending plane, and the first inferior rail may be spaced apart from the laterally extending plane by a second rail distance further than the second inferior rail is spaced apart from the laterally extending plane. In addition, the first rail distance may be no less than a width of each of the first and second inferior rails, and the second rail distance may be no less than a width of each of the first and second superior rails. The first and second superior rails and the first and second inferior rails may thus be sized and disposed on their respective components to allow the first and second superior rails to interdigitate with the first and second inferior rails.

The first superior rail may extend width wise from a boundary on one side of the superior component, and the second superior rail may extend away from the laterally extending plane by a boundary distance short of a boundary on the other side of the superior component, the boundary distance of sufficient extent to receive a rail of the inferior component and without the received rail extending substantially beyond the boundary on the other side of the superior component. Likewise, the first inferior rail may extend width wise from a boundary on one side of the inferior component, and the second inferior rail may extend away from the laterally extending plane by a boundary distance short of a boundary on the other side of the inferior component, the boundary distance of sufficient extent to receive a rail of the superior component and without the received rail extending substantially beyond the boundary on the other side of the inferior component.

The superior and inferior components may be substantially the same at least in respect of disposition and size of their respective rails. Furthermore, the superior and inferior components may be substantially the same. The superior and inferior components may therefore be exchanged whereby the superior component can function as the inferior component and vice-versa. Having superior and inferior components which are substantially the same as each other may reduce manufacturing costs. Aside from this, the burden is reduced on the surgeon because the surgeon does not need to take care to distinguish the superior and inferior components from each other.

Each rail of the superior and inferior components may define a channel which extends along the rail and which faces towards a laterally extending plane of the respective component which bisects the component. The channel of the first superior rail may therefore face the channel of the second superior rail, and the channel of the first inferior rail may therefore face the channel of the second inferior rail. In use, each channel slidably receives a respective elongate protrusion on the core component during insertion of the core component between the superior and inferior components. Each channel may be open at a first channel end and closed at a second channel end. The openings at the first channel end of the first and second superior and inferior rails may be at a first end of the component which first receives the core component upon insertion of the core component between the superior and inferior components. The closed nature of the second channel ends of the first and second superior and inferior rails at a second, opposite end of the superior/inferior component may arrest movement of the core component relative to the superior/inferior component upon full insertion of the core component between the superior and inferior components, i.e. when the core component is in registration with the superior and inferior components.

The interdigitated nature of the rails of the superior and inferior components may allow the superior and inferior components to rotate relative to each other while maintaining some extent of interdigitation of their respective rails. For example, the superior and inferior components may be separated at their first ends while there is less or perhaps even substantially no separation between the superior and inferior components at their second ends. Relative rotation of the superior and inferior components may occur when the core component is inserted between the superior and inferior components from their first ends.

Each of the superior and inferior components may be configured to provide for ease of their relative rotation about their second ends. More specifically, a plurality of interdigitating projections may extend up from the superior component bottom side and from the inferior component top side. There may be two interdigitating projections extending up from the respective side. The interdigitating projections may be spaced apart in a direction orthogonal to the lateral direction. The interdigitating projections may be of relatively short length whereby each projection extends from the second end a short way across the respective side towards the first end (i.e. in the lateral direction). Each projection may extend in the lateral direction across no more than 20 percent of the respective side.

As per the rails of the superior and inferior components, first and second interdigitating projections may be non-equidistantly spaced from a laterally extending plane which bisects the respective one of the superior and inferior components. Further features of the first and second interdigitating projections of the superior and inferior components in respect of their disposition and size may be as defined above with reference to the rails of the superior and inferior components. The first and second interdigitating projections of the superior component may thus interdigitate with the first and second interdigitating projections of the inferior component.

The first interdigitating projection may be longer in the lateral direction than the second interdigitating projection to present a barrier to advancement of the core component between the superior and inferior components. A leading end of the first interdigitating projection (i.e. the end of the first interdigitating projection closer to the first end of the superior/inferior component) may be received in a correspondingly shaped recess defined by the core component.

The rails of the superior and inferior components may extend from their second ends towards their first ends. The rails of each of the superior and inferior components may extend across at least 90 percent of the length (i.e. in the lateral direction) of the component. A portion of the end of each rail towards the first end of the component may slope down to the respective one of the superior component bottom side and the inferior component top side to thereby provide for ease of insertion of the core component between the superior and inferior components.

Each rail of the superior and inferior components defines a distal surface at a distal end thereof. The distal surface of the first superior rail bears against the inferior component top side and the first inferior rail bears against the superior component bottom side when the rails of the superior and inferior components interdigitate.

The superior and inferior components are configured such that the superior component top side and the inferior component bottom side are further apart from each other at the first ends of the superior and inferior components than the second ends of the superior and inferior components. There may be a progressive change in this regard between the first and second ends. According to one approach, a part of the inferior component top side against which the first superior rail bears may slope between the first and second ends, and a part of the superior component bottom side against which the first inferior rail bears may slope between the first and second ends when the rails of the superior and inferior components interdigitate. Alternatively or in addition and according to another approach, the height of each of the first superior rail and the first inferior rail may increase progressively towards the second end whereby the distal surfaces of the first superior rail and the first inferior rail are sloped. Having the superior component top side and the inferior component bottom side further apart from each other at the first ends of the superior and inferior components than the second ends of the superior and inferior components may provide for distraction at the second end. Aside from this, lower height of the interdigitated superior and inferior components at the second end may provide for ease of introduction of the interdigitated superior and inferior components into the intervertebral space.

The core component may have a core component top side and a core component bottom side. The core component top side and the core component bottom side may be inclined to each other. The core component may therefore have the form of a wedge. The core component top side and the core component bottom side may not meet at an acute angle whereby the core component has the form of a frustum of a wedge. Typically, the core component top side and the core component bottom side may be inclined to each other in a direction orthogonal to the lateral direction, to thereby provide for spinal correction in the sagittal plane. Alternatively, the core component top side and the core component bottom side may be inclined to each other in the lateral direction to thereby provide for spinal correction in the coronal plane.

A wedge shaped core component when inserted between the superior and inferior components causes relative inclination of the superior component top side and the inferior component bottom side. Extent of inclination of the superior component top side and the inferior component bottom side may be determined by selection from a plurality of core components having core component top and bottom sides of different relative inclinations.

As described above, each channel of the superior and inferior components may slidably receive a respective elongate protrusion of the core component during insertion of the core component between the superior and inferior components. The core component may define first and second elongate protrusions at each of the core component top side and the core component bottom side. The first and second elongate protrusions may extend along their length in the lateral direction and may protrude from oppositely directed sides of the core component at a respective one of the core component top side and the core component bottom side. The first and second elongate protrusions may define in part a respective one of the core component top side and the core component bottom side. Each elongate protrusion may be shaped and sized to be slidably received in a respective channel and more specifically to be a snug fit in the channel while allowing for ease of movement of the protrusions in their respective channels.

As described above, the first and second superior rails may be non-equidistantly spaced from a laterally extending plane which bisects the superior component, and the first and second inferior rails may be non-equidistantly spaced from a laterally extending plane which bisects the inferior component. Therefore, the first and second elongate protrusions at the core component top side may be offset from the first and second elongate protrusions at the core component bottom side. The relative disposition of the first and second elongate protrusions at the core component top and bottom sides may provide for registration of each of the first and second elongate protrusions with its respective rail defined channel.

Each of the first and second elongate protrusions may define along its length a sprung arm. The sprung arm may define a detent at its distal end. The detent may inter-engage with a correspondingly shaped formation on the respective superior or inferior component to thereby present resistance to the core component being ejected from between the superior and inferior components.

A leading end of the core component, i.e. the end of the core component received first between the superior and inferior components, may have rounded corners. Rounded corners at the leading edge may provide for ease of insertion of the core component between the inferior and superior components and also ease of movement of adjacent nerve structures with reduced risk of damage to the adjacent nerve structures.

Alternatively or in addition, upper and lower surfaces at the leading (or first) end of the core component may be inclined to each other whereby the leading end narrows towards its distal end. Each of the upper and lower surfaces may be inclined to a transverse plane that bisects the core component into upper and lower halves. The transverse plane may be orthogonal to each of the coronal and sagittal planes. The leading end may therefore be wedge shaped. The leading end with inclined upper and lower surfaces may extend towards the trailing (or second) end of the core component by no more than 10 percent of the length of the core component. The leading end may lack the first and second elongate protrusions. Having a leading end which is structured in this fashion may provide for ease of introduction of the core component between the superior and inferior components. More specifically, the leading end may provide for proper seating of the core component relative to the superior and inferior components before the first and second elongate protrusions start to engage with their respective channels. This may minimise risk of jamming when the first and second elongate protrusions engage with their respective channels.

When the core component is wedge shaped in a direction orthogonal to the lateral direction, the upper and lower surfaces of the leading end may slope in each of the lateral direction and the direction orthogonal to the lateral direction. This may provide for distraction of the superior and inferior components and relative rotation of the superior and inferior components about a main axis of the core component, the main axis extending in the lateral direction. Distraction and relative rotation may position the superior and inferior components for engagement of the first and second elongate protrusions with their respective channels with reduced risk of jamming.

A modular LLIF device having separate inferior and superior components and core component means the components may be introduced to the intervertebral space more gently compared with known single piece intervertebral fusion devices which often need to be hammered into place. Such a less gentle insertion process may damage the intervertebral fusion device, may increase time required for the LLIF device to settle in the intervertebral space, and may result in trauma to vertebral bodies or adjacent soft tissues including neural structures. On the subject of trauma, a device that is hammered into place is liable to create microfractures in the vertebrae which could lead to subsidence of the device into the host bone. Furthermore, having separate components and in particular a core component separate to the inferior and superior components allows for differences in dimensions of intervertebral spaces, differences in angle between the adjacent vertebrae that define the intervertebral space, and differences in degree of spinal alignment and/or correction. Each of the superior component, the inferior component and the core component may be integrally formed.

Each of the superior and inferior components may have the form of a plate, albeit a plate having structures thereon including the rails that provide for mechanical engagement with the core component, whereby it is thin relative to its width and depth. At least one of the superior component top side and the inferior component bottom side may be shaped in the coronal and/or sagittal planes, for example domed, to enhance fit and contact with the adjacent vertebrae.

Each of the superior component, the inferior component, and the core component may be oblong. Each of the superior component, the inferior component, and the core component may be a rounded rectangle and more specifically a rectangle with rounded corners. Rounded corners may provide for ease of introduction into the intervertebral space. The components may be longer than wider in the lateral direction.

At least one of the superior component top side and the inferior component bottom side may be configured to provide for fusion. For example, the top or bottom side may comprise formations, such as protrusions, which, in use, engage with the bone of the vertebra. By way of another example, the top and/or bottom side may define apertures for passage of bone graft material therethrough from an interior of the intervertebral fusion device. By way of a further example, the top or bottom side may have a coating thereon or impregnation therein. The coating or impregnation may comprise material that provides for bone adhesion and/or bone formation to encourage bone to grow up to and bond onto the LLIF device to thereby provide long term stable attachment. One or more known coatings may be used, such as porous mesh, tricalcium phosphate (TCP), hydroxyapatite (HA) or bone morphogenetic protein (BMP).

At least one of the superior component, the core component and the inferior component may be formed from a metal, such as titanium, or a metal alloy, such as stainless steel, Ti6Al4V, CoCr or nitinol. Nitinol may be useful in respect of cooperating parts of the superior component, the core component and the inferior component. At least one of the superior component, the core component and the inferior component may be formed from a plastics material and more specifically a thermoplastic polymer, such as PEEK or carbon reinforced PEEK. In forms of the invention, the core component may be formed by 3D printing whereby the core component has the form of a 3D lattice. The aforementioned materials may be used to form the core component by way of 3D printing.

Each of the superior component top side and the inferior component bottom side may have a length of between 40 mm and 60 mm and a width of between 15 mm and 25 mm.

The core component may have a length of between 38 mm and 58 mm and a width of between 15 mm and 25 mm.

When assembled, i.e. when the core component is fully received between the superior and inferior components, the LLIF device may have a height between 6 mm and 12 mm. The height may be measured at the back of the LLIF device, i.e. the part of the LLIF device first received in the intervertebral space upon insertion. The LLIF device may have a corrective angle of between 0 degrees and 40 degrees.

At least one of the superior and inferior components may be sloped in the coronal direction to thereby provide for spinal correction in the coronal direction. Furthermore, the core component may not be sloped in the coronal direction whereby spinal correction in the coronal direction is accomplished by at least one of the superior and inferior components. The core component top side and a core component bottom side may therefore be inclined to each other in the coronal direction.

According to a second aspect of the present invention there is provided a method (not claimed) of installing a modular lateral lumbar interbody fusion (LLIF) device in an intervertebral space between first and second adjacent vertebrae, the LLIF device comprising a superior component having a superior component top side and a superior component bottom side, an inferior component having an inferior component top side and an inferior component bottom side, and a core component, the method comprising:
positioning the superior and inferior components relative to each other such that the superior component bottom side and the inferior component top side oppose each other;
positioning the superior and inferior components in the intervertebral space such that the superior component top side abuts against the first vertebra and the inferior component bottom side abuts against the second vertebra; and
inserting the core component between the superior and inferior components whereby a separation between the superior and inferior components and hence height of the **LLIF** device are determined,
wherein first and second superior rails protrude from the superior component bottom side, the first and second superior rails extending in the lateral direction, the first and second superior rails substantially parallel to each other and spaced apart from each other in a direction substantially orthogonal to the lateral direction,
wherein first and second inferior rails protrude from the inferior component top side, the first and second inferior rails extending in the lateral direction, the first and second inferior rails substantially parallel to each other and spaced apart from each other in a direction substantially orthogonal to the lateral direction,
wherein the first and second superior rails and the first and second inferior rails are disposed on their respective component such that the first and second superior rails interdigitate with the first and second inferior rails when the superior and inferior components are in registration and abut against each other before insertion of the core component between the superior and inferior components, and
wherein the core component is configured to inter-engage with each of the first and second superior rails and each of the first and second inferior rails during insertion of the core component between the superior and inferior components.

The intervertebral fusion device comprises a superior component having a superior component top side and a superior component bottom side, an inferior component having an inferior component top side and an inferior component bottom side, and a core component. The method of installing the LLIF device in an intervertebral space between first and second adjacent vertebrae comprises positioning the superior component and the inferior component relative to each other such that the superior component bottom side and the inferior component top side oppose each other.

The method further comprises positioning the superior component and the inferior component in the intervertebral space such that the superior component top side abuts against the first vertebra and the inferior component bottom side abuts against the second vertebra. Typically, the step of positioning the superior component and the inferior component relative to each other may be carried out before the following step. For example, the superior and inferior components may be mounted on an inserter whereby they are positioned relative to each other such that the superior component bottom side and the inferior component top side oppose each other. The inserter may then be used to position the superior component and the inferior component in the intervertebral space. The method then comprises inserting the core component between the superior and inferior components whereby a separation between the superior and inferior components and hence height of the LLIF device are determined when the LLIF device is in the intervertebral space. Typically, the core component may be inserted between the superior and inferior components by a core loader, which may be comprised in the inserter.

First and second superior rails protrude from the superior component bottom side. The first and second superior rails extend in the lateral direction substantially parallel to each other. The first and second superior rails are spaced apart from each other in a direction substantially orthogonal to the lateral direction. The inferior component top side is likewise structured by way of first and second inferior rails which protrude from the inferior component top side. The first and second inferior rails extend in the lateral direction substantially parallel to each other. The first and second inferior rails are spaced apart from each other in a direction substantially orthogonal to the lateral direction. The first and second superior rails and the first and second inferior rails are disposed on their respective component such that the first and second superior rails interdigitate with the first and second inferior rails when the superior and inferior components are in registration and abut against each other before insertion of the core component between the superior and inferior components. The core component is configured to inter-engage with each of the first and second superior rails and each of the first and second inferior rails during insertion of the core component between the superior and inferior components.

Further embodiments of the second aspect of the present invention may comprise one or more features of the first aspect of the present invention.

The present inventors have appreciated features of the leading end of the core component to be of wider applicability than hitherto described. Therefore, and according to a third aspect of the present invention, there is provided a modular interbody fusion device comprising:
a superior component having a superior component top side and a superior component bottom side, the superior component configured to be received in an intervertebral space between first and second vertebrae whereby the superior component top side abuts against the first vertebra;
an inferior component having an inferior component top side and an inferior component bottom side, the inferior component configured to be received in the intervertebral space between the first and second vertebrae whereby the inferior component bottom side abuts against the second vertebra, the superior component bottom side and the inferior component top side opposing each other when the superior and inferior components are received in the intervertebral space; and
a core component having a core component top side and a core component bottom side, the core component configured for insertion between the superior and inferior components whereby the core component top side faces the superior component bottom side and the core component bottom side faces the inferior component top side and a separation between the superior and inferior components and hence height of the interbody fusion device are determined,
wherein the superior component and the core component define structures which slidably inter-engage the core component with the superior component during insertion of the core component between the superior and inferior components,
wherein the inferior component and the core component define structures which slidably inter-engage the core component with the inferior component during insertion of the core component between the superior and inferior components, and
wherein the core component comprises a main core body and a leading portion extending from the main core body at an end of the core component first received between the superior and inferior components upon insertion of the core component between the superior and inferior components, parts of the core component top and bottom sides defined by the leading portion inclined to each other to greater extent than parts of the core component top and bottom sides defined by the main core body, whereby the leading end narrows towards its distal end.

The modular interbody fusion device comprises three main components, namely a superior component, an inferior component and a core component. The interbody fusion device is modular. In other words, the superior and inferior components and the core component are separate components. The superior and inferior components are therefore not attached to each other before assembly of the interbody fusion device. The superior and inferior components may be attached to each other solely by the core component when the interbody fusion device has been assembled. The modular interbody fusion device may be a posterior lumbar interbody fusion (PLIF), anterior lumbar interbody fusion (ALIF), a lateral lumbar interbody fusion (LLIF), or an oblique lumbar interbody fusion (OLIF) device.

In use, the superior and inferior components are placed in an intervertebral space between first and second vertebrae formed by at least partial removal of a problematic intervertebral disc. The superior component has a superior component top side and a superior component bottom side with the superior component placed in the intervertebral space such that the superior component top side faces the first vertebra or what might remain of a partially removed intervertebral disc. The inferior component has an inferior component top side and an inferior component bottom side with the inferior component placed in the intervertebral space such that the inferior component bottom side faces the second vertebra or what might remain of a partially removed intervertebral disc. The superior component bottom side and the inferior component top side oppose each other when the superior and inferior components are received in the intervertebral space. The superior and inferior components may be in registration with each other when in the intervertebral space and more specifically when the core component is fully inserted between the superior and inferior components, as described below. Introduction of the superior and inferior components without the core component into the intervertebral space allows for them to be moved around the adjacent nerve structures and with reduced requirement for retraction of the adjacent nerve structures. Risk of damage to the adjacent nerve structures is therefore reduced.

The core component is configured for insertion between the superior and inferior components. Upon insertion the core component determines a separation between the superior and inferior components and hence a height of the **LLIF** device with the superior component top side abutting against the first vertebra or what remains of the partially removed intervertebral disc and with the inferior component bottom side abutting against the second vertebra or what remains of the partially removed intervertebral disc. When the core component has been inserted between the superior and inferior components, the core component top side of the core component faces the superior component bottom side and the core component bottom side of the core component faces the inferior component top side. Differing heights of LLIF device may be provided by selection from plural core components of different height.

The superior component and the core component define structures which slidably inter-engage the core component with the superior component during insertion of the core component between the superior and inferior components. Further to this, the inferior component and the core component define structures which slidably inter-engage the core component with the inferior component during insertion of the core component between the superior and inferior components. The structures therefore provide for slidable inter-engagement of the core component with each of the superior and inferior components when the core component is inserted between the superior and inferior components. Also, the structures provide for inter-engagement of the core component with each of the superior and inferior components when the interbody fusion device has been assembled, i.e. when the core component has been inserted fully between the superior and inferior components.

The core component comprises a main core body and a leading portion extending from the main core body at an end of the core component first received between the superior and inferior components upon insertion of the core component between the superior and inferior components. Parts of the core component top and bottom sides defined by the leading portion are inclined to each other to greater extent than the core component top and bottom sides defined by the main core body, whereby the leading end narrows towards its distal end. The parts of the core component top and bottom sides defined by the leading portion may be tapered. Where the core component is wedge shaped, the core component top and bottom sides defined by the main core body may be inclined to each other and more specifically may be inclined such that they are closer together towards the leading portion. In other forms, the core component top and bottom sides defined by the main core body may not be inclined to each other.

A modular interbody fusion device which has a core component having a leading portion as described above may provide for ease of assembly of the modular interbody fusion device. More specifically, the leading portion may provide for ease of insertion of the core component between the superior and inferior components. Aside from this, the leading portion may move the superior and inferior components relative to each other whereby they are more properly relatively disposed for subsequent inter-engagement of the structures on the core component with the structures on the superior and inferior components and with reduced risk of jamming during insertion of the core component.

Each of the core component top and bottom sides may be inclined to a transverse plane that bisects the core component into upper and lower halves. The transverse plane may be orthogonal to each of the coronal and sagittal planes. The leading end may therefore be wedge shaped. The leading end with inclined core component top and bottom sides may extend towards the trailing end of the core component by no more than 10 percent of the length of the core component. The leading end may lack the structures that provide for inter-engagement with the superior and inferior components.

When the core component is wedge shaped in a direction orthogonal to the lateral direction, the core component top and bottom sides of the leading end may slope in each of the lateral direction and a direction orthogonal to the lateral direction. This may provide for distraction of the superior and inferior components and relative rotation of the superior and inferior components about a main axis of the core component, the main axis extending in the lateral direction. Distraction and relative rotation may position the superior and inferior components for engagement of the first and second elongate protrusions with their respective channels with reduced risk of jamming.

Further embodiments of the third aspect of the present invention may comprise one or more features of the first aspect of the present invention.

According to a fourth aspect of the present invention there is provided a method (not claimed) of installing a modular interbody fusion device in an intervertebral space between first and second adjacent vertebrae, the interbody fusion device comprising a superior component having a superior component top side and a superior component bottom side, an inferior component having an inferior component top side and an inferior component bottom side, and a core component, the method comprising:
positioning the superior component and the inferior component relative to each other such that the superior component bottom side and the inferior component top side oppose each other;
positioning the superior component and the inferior component in the intervertebral space such that the superior component top side abuts against the first vertebra and the inferior component bottom side abuts against the second vertebra; and
inserting the core component between the superior and inferior components whereby the core component top side faces the superior component bottom side and the core bottom side faces the inferior component top side and a separation between the superior and inferior components and hence height of the interbody fusion device are determined,
wherein the superior component and the core component define structures which slidably inter-engage the core component with the superior component during insertion of the core component between the superior and inferior components,
wherein the inferior component and the core component define structures which slidably inter-engage the core component with the inferior component during insertion of the core component between the superior and inferior components, and
wherein the core component comprises a main core body and a leading portion extending from the main core body at an end of the core component first received between the superior and inferior components upon insertion of the core component between the superior and inferior components, parts of the core component top and bottom sides defined by the leading portion inclined to each other to greater extent than parts of the core component top and bottom sides defined by the main core body, whereby the leading end narrows towards its distal end.

Embodiments of the fourth aspect of the present invention may comprise one or more features of the first or third aspect of the present invention

### Brief Description of Drawings

Further features and advantages of the present invention will become apparent from the following specific description, which is given by way of example only and with reference to the accompanying drawings, in which:
Figure 1A is a first perspective view of a superior/inferior component of a modular interbody fusion device according to the present invention;
Figure 1B is a second perspective view of the superior/inferior component of Figure 1A;
Figure 1C is a view of a first end of the superior/inferior component of Figure 1A;
Figure 1D is a side view of the superior/inferior component of Figure 1A;
Figure 2A is a perspective view of the superior and inferior components when in an interdigitating condition;
Figure 2B is a view of the second ends of the superior and inferior components when in an interdigitating condition;
Figure 2C is a side view of the superior and inferior components when in an interdigitating condition;
Figure 2D is a view in cross-section through A-A in Figure 2C;
Figure 2E is a view in cross-section through B-B in Figure 2C;
Figure 2F is a view in cross-section through C-C in Figure 2C;
Figure 3A is a perspective view of a core component of the modular interbody fusion device according to the present invention;
Figure 3B is a view of a trailing end of the core component of Figure 3A;
Figure 3C is a side view of the core component of Figure 3A;
Figure 3D is a view of a leading end of the core component of Figure 3A;
Figure 3E is a top view of the core component of Figure 3A;
Figure 4A shows the core component of Figures 3A to 3E before insertion between the superior and inferior components of Figures 1A to 2F;
Figure 4B shows the core component of Figure 4A after insertion between the superior and inferior components of Figure 4A; and
Figure 4C is an end view of the assembled modular interbody fusion device shown in Figure 4B.

### Description of Embodiments

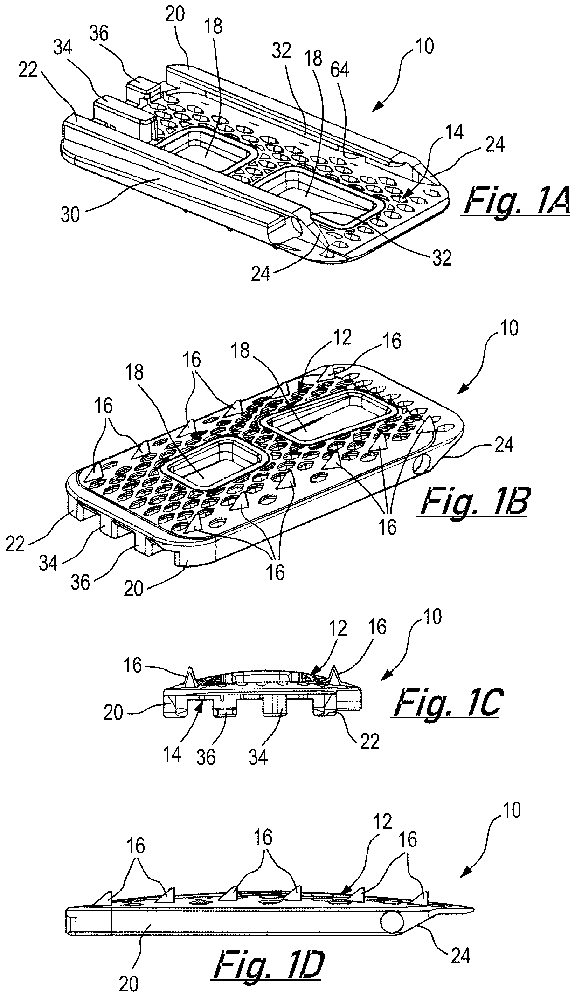

A superior component 10 of a modular lateral lumbar interbody fusion (LLIF) device according to the present invention is shown in Figures 1A to 1D. The superior and inferior components of the LLIF device are substantially identical whereby the present description of the superior component 10 made with reference to Figures 1A to 1D should also be treated as description of the inferior component 10. Figures 1A and 1B are first and second perspective views respectively of the superior component 10. Figure 1C is a view of a first end of the superior component 10 and Figure 1D is a side view of the superior component 10. The superior component 10 has a superior component top side 12 (which is an inferior component bottom side of the inferior component) and a superior component bottom side 14 (which is an inferior component top side of the inferior component). Figure 1A is a perspective view from one corner and above the superior component bottom side 14 and Figure 1B is a perspective view from one corner and above the superior component top side 12. In respect of the inferior component, Figure 1A is a perspective view from one corner and above the inferior component top side 14 and Figure 1B is a perspective view from one corner and above the inferior component bottom side 12.

As can be appreciated from Figures 1A and 1B, the superior and inferior components 10 are rounded rectangles when viewed from above or below. The rounded corners of the superior and inferior components 10 provides for ease of their insertion into the patient's body. A lateral direction of the superior/inferior component 10 extends in the same direction as a longitudinal axis of the superior/inferior component 10. The lateral direction is the direction in which the superior and inferior components 10 are introduced into the patient's body and the direction in which the core component, which is described below, is inserted between the superior and inferior components. Each of the superior and inferior components 10 has the form of a plate, albeit a plate having structures thereon, as described below, whereby it is thin relative to its width and depth. As can be seen from Figures 1C and 1D, the superior component top side/inferior component bottom side 12 is slightly domed in the coronal and sagittal planes to enhance fit and contact with the adjacent vertebrae. Further to this, the superior component top side/inferior component bottom side 12 defines a plurality of spaced apart small protrusions 16, which, in use, engage with the bone of the vertebra. The superior component top side/inferior component bottom side 12 also defines two large bone graft apertures 18 and numerous small bone graft apertures (not indicated with a reference numeral but clearly shown in Figures 1A to 1D) for passage of bone graft material therethrough from an interior of the LLIF device. Further to this, the superior component top side/inferior component bottom side 12 has a coating thereon or impregnation therein. The coating or impregnation comprises material that promotes bone adhesion and/or bone formation to encourage bone to grow up to and bond onto the LLIF device to thereby provide long term stable attachment. One or more known coatings are used, such as porous mesh, tricalcium phosphate (TCP), hydroxyapatite (HA) or bone morphogenetic protein (BMP).

First 20 and second 22 superior rails protrude from the superior component bottom side 14. The first and second superior rails 20, 22 of the superior component 10 correspond respectively to first and second inferior rails of the inferior component. The first and second superior rails 20, 22 are straight and parallel to each other. The first and second superior rails 20, 22 extend along their length across the superior component bottom side 14 in the lateral direction and are spaced apart from each other in a direction orthogonal to the lateral direction.

The first superior rail 20 extends width wise from a boundary on one side of the superior component 10, and the second superior rail 22 extends width wise in a direction away from a laterally extending plane, which bisects the superior component, by a boundary distance short of a boundary on the other side of the superior component. The boundary distance is of sufficient width to receive the first inferior rail of the inferior component and without the first inferior rail extending substantially beyond the boundary on the other side of the superior component. In view of the inferior component being substantially identical to the superior component, the first inferior rail extends width wise from a boundary on one side of the inferior component, and the second inferior rail extends width wise in a direction away from the laterally extending plane by a boundary distance short of a boundary on the other side of the inferior component. The boundary distance is of sufficient extent to receive first superior rail of the superior component and without the received first superior rail extending substantially beyond the boundary on the other side of the inferior component.

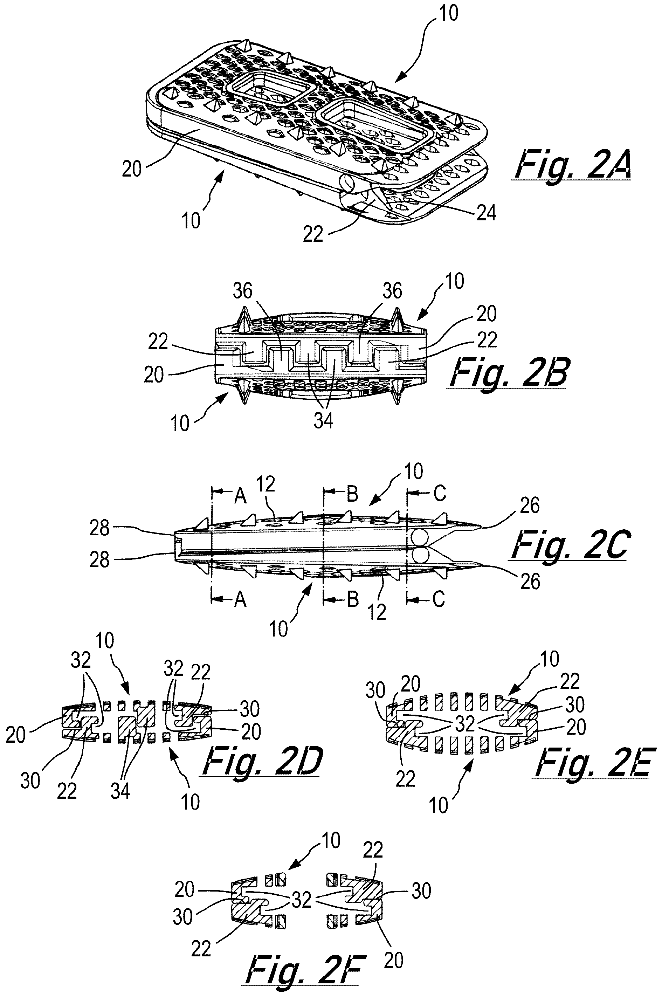

The first and second superior rails 20, 22 are thus non-equidistantly spaced from the laterally extending plane, and the first and second inferior rails are non-equidistantly spaced from the laterally extending plane. Furthermore, the first and second superior rails 20, 22 and the first and second inferior rails are thus sized and disposed on their respective component 10 to allow the first and second superior rails 20, 22 to interdigitate with the first and second inferior rails when the superior and inferior components are in registration and when they abut against each other, as shown in Figures 2A and 2B. Figure 2A is a perspective view of the superior and inferior components when in the interdigitating condition and Figure 2B is a view of the second ends of the superior and inferior components when in the interdigitating condition. Referring to Figure 1A, each of the first and second superior rails 20, 22 extends along at least 90 percent of the length (i.e. in the lateral direction) of the superior component 10. One end of each of the first and second superior rails 20, 22 is at the second end of the superior component 10 and the other end of the first and second superior rails is short of the first end of the superior component. The first end of the superior/inferior component is the end at which the core component is first received between the superior and inferior components. A portion 24 of the end of each of the first and second superior rails 20, 22 towards the first end of the superior component 10 slopes down to the superior component bottom side 14 to thereby provide for proper insertion of the core component between the superior and inferior components at their first ends. Each of the first and second superior rails 20, 22 defines a planar distal surface at a distal end thereof.

Referring now to Figure 2C, the superior and inferior components are configured such that the superior component top side 12 and the inferior component bottom side 12 are further apart from each other at the first ends 26 of the superior and inferior components 10 than the second ends 28 of the superior and inferior components when the superior and inferior components are in the interdigitated condition. Figure 2C is a side view of the superior and inferior components when in the interdigitating condition. Figure 2D is a view in cross-section through A-A in Figure 2C, Figure 2E is a view in cross-section through B-B in Figure 2C, and Figure 2F is a view in cross-section through C-C in Figure 2C. Referring to Figure 2D, Figure 2E and Figure 2F in turn, the part 30 of the superior component bottom side 14 against which the first inferior rail 20 bears and the part 30 of the inferior component top side 14 against which the first superior rail 20 bears both increase in height from the second end 28 to the first end 26. The increase in height is progressive from the second end 28 to the first end 26. The increase in height of the parts 30 of the superior component bottom side 14 and the inferior component top side 14 provides for the superior component top side 12 and the inferior component bottom side 12 being further apart from each other at the first ends 26 of the superior and inferior components 10 than the second ends 28 of the superior and inferior components. This provides for distraction of the superior and inferior components 10 at the second end 28. Aside from this, lower height of the interdigitated superior and inferior components 10 at the second end 28 provides for ease of introduction of the interdigitated superior and inferior components into the intervertebral space.

Referring back to Figure 1A, each of the first and second superior rails 20, 22 defines a channel 32 in an interior side thereof. The channel 32 of the first superior rail 20 faces the channel 32 of the second superior rail 22. Correspondingly, the channel 32 of the first inferior rail 20 faces the channel 32 of the second inferior rail 22. Each channel 32 slidably receives a respective elongate protrusion on the core component during insertion of the core component between the superior and inferior components 10, as described further below. Each channel 32 is open at a first channel end and closed at a second channel end. The channel openings at the first channel end of the first and second superior and inferior rails 20, 22 are at the first end 26 of the superior/inferior component 10 which first receives the core component upon insertion of the core component between the superior and inferior components. The closed nature of the second channel ends of the first and second superior and inferior rails 20, 22 at the second end 28 of the superior/inferior component 10 arrests movement of the core component relative to the superior/inferior component upon full insertion of the core component between the superior and inferior components, i.e. when the core component is in registration with the superior and inferior components.

Referring to Figure 1A, first 34 and second 36 interdigitating projections extend up from the superior component bottom side 14/inferior component top side 14. The first and second interdigitating projections 34, 36 are the same width in a direction orthogonal to the lateral direction. The first and second interdigitating projections 34, 36 are spaced apart in the direction orthogonal to the lateral direction and are straight along their length in the lateral direction whereby they are parallel. The first and second interdigitating projections 34, 36 are offset from the laterally extending plane by distance a little more than the width of each of the first and second interdigitating projections. The first and second interdigitating projections 34, 36 thus interdigitate in the same fashion as interdigitation of the rails 20, 22 when the superior and inferior components 10 are in registration and abut each other, and as shown in Figure 2B. The first and second interdigitating projections 34, 36 are of relatively short length whereby each projection extends in the lateral direction from the second end a short way across the superior component bottom side 14/inferior component top side 14. Each projection 34, 36 extends in the lateral direction across no more than 20 percent of the superior component bottom side 14/inferior component top side 14. As can be seen from Figure 1A, the first interdigitating projection 34 is longer in the lateral direction than the second interdigitating projection 36. In use, the end of the first interdigitating projection 34 further away from the second end 28, i.e. the leading end, presents a barrier to advancement of the core component between the superior and inferior components. The leading end of each first interdigitating projection 34 is received in a correspondingly shaped projection recess defined by the core component.

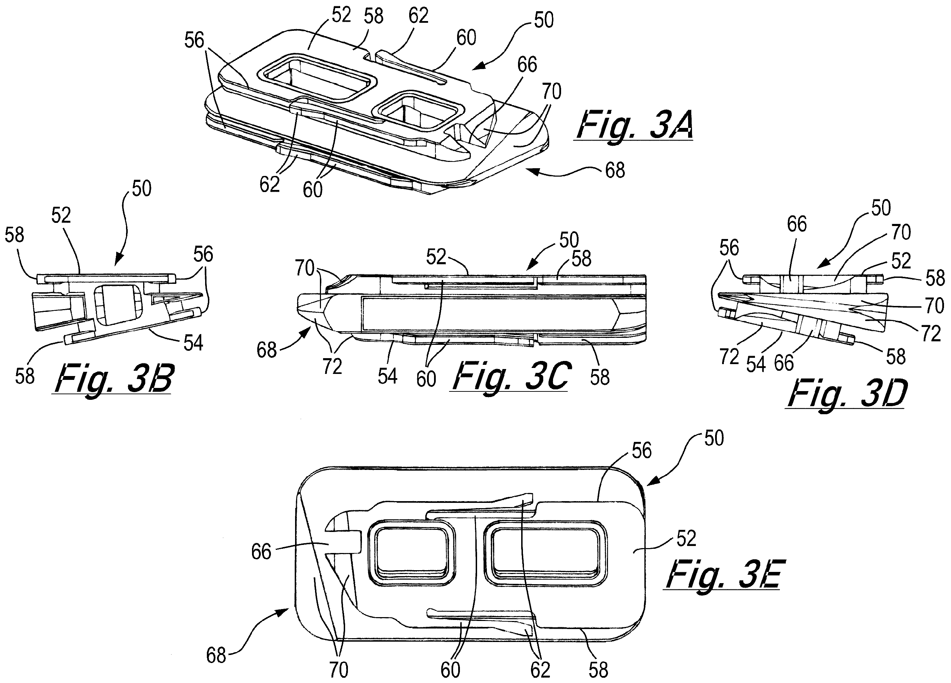

A core component 50 of the modular lateral lumbar interbody fusion (LLIF) device according to the present invention is shown in Figures 3A to 3E. Figure 3A is a perspective view of the core component 50, Figure 3B is a view of a trailing end of the core component, Figure 3C is a side view of the core component, Figure 3D is a view of a leading end of the core component, and Figure 3E is a top view of the core component.

The core component 50 has a core component top side 52 and a core component bottom side 54. As can be seen from Figure 3A and more particularly Figures 3B and 3C, the core component top side 52 and the core component bottom side 54 are inclined to each other in a direction orthogonal to the lateral direction, whereby the core component has the general form of a frustum of a wedge. Insertion of the core component 50 between the superior and inferior components 10 causes corresponding relative inclination of the superior component top side 12 and the inferior component bottom side 12. Extent of inclination of the superior component top side and the inferior component bottom side is determined by selection from a plurality of core components 50 having core component top and bottom sides 52, 54 of different relative inclinations. In forms of the invention, the superior component top side and the inferior component bottom side are not inclined to each other whereby the LLIF device provides no correction angle.

The core component 50 defines first 56 and second 58 elongate protrusions on each of the core component top side 52 and the core component bottom side 54. First 56 and second 58 elongate protrusions extend along their length in the lateral direction at opposite sides of the core component top side 52 such that the protrusions constitute part of the core component top side. First 56 and second 58 elongate protrusions extend along their length in the lateral direction at opposite sides of the core component bottom side 54 such that a side of the protrusions constitute part of the core component bottom side. The first and second 56, 58 elongate protrusions protrude in opposite directions from a respective side of the core component. Each elongate protrusion 56, 58 is shaped and sized to be slidably received in a respective channel 32 of a respective one of the superior and inferior components 10 during insertion of the core component between the superior and inferior components. As described above, the first and second superior/inferior rails 20, 22 are non-equidistantly spaced from the laterally extending plane. As shown in Figures 3B and 3C, the first and second elongate protrusions 56, 58 at the core component top side 52 are offset from the first and second elongate protrusions 56, 58 at the core component bottom side 54. This offsetting of the first and second elongate protrusions 56, 58 provides for registration of each elongate protrusion with its respective rail defined channel 32.

Referring now to Figures 3A and 3E, each of the first and second elongate protrusions 56, 58 defines along its length an integrally formed sprung arm 60. Each sprung arm 60 defines a protrusion 62 at its distal end. Each protrusion 62 interengages with a correspondingly shaped recess 64 formed on the respective superior or inferior component 10 to thereby present resistance to ejection of the core component 50 from between the superior and inferior components when the core component has been fully received between the superior and inferior components. Each of the core component top side 52 and the core component bottom side 54 define a projection recess 66 towards the leading end 68 of the core component 50. Each projection recess 66 extends away from the leading end 68 in the lateral direction and is sized and shaped to receive a respective one of the first interdigitating projections 34 on the superior and inferior components 10. As described above, the end of the first interdigitating projections 34 abut against the bases of the projection recesses 66 to present a barrier to advancement of the core component between the superior and inferior components when the core component is fully received between the superior and inferior components.

As can be seen from Figure 3E, the leading end 68 of the core component 50, i.e. the end of the core component received first between the superior and inferior components 10, has rounded corners. The rounded corners provide for ease of insertion of the core component 50 between the inferior and superior components 10 and also ease of movement of adjacent nerve structures with reduced risk of damage to the adjacent nerve structures when the core component is introduced into the patient's body. Further to this, the leading end 68 is shaped such that it tapers in the lateral direction towards its distal end. The taper is formed by upper 70 and lower 72 surfaces that are inclined to each other. The leading end 68 lacks the first and second elongate protrusions 56, 58. In view of the core component 50 being wedge shaped in a direction orthogonal to the lateral direction, the upper and lower surfaces 70, 72 slope in each of the lateral direction and the direction orthogonal to the lateral direction. This can be seen in the view of Figure 3C. The leading end 68 provides for ease of introduction of the core component 50 between the superior and inferior components 10 by providing for proper seating of the core component relative to the superior and inferior components before the first and second elongate protrusions 56, 58 start to engage with their respective channels 32 to thereby minimise risk of jamming when the first and second elongate protrusions engage with their respective channels. Considering this further, the leading end 68 distracts the superior and inferior components 10 and causes their relative rotation about a main axis of the core component, the main axis extending in the lateral direction.

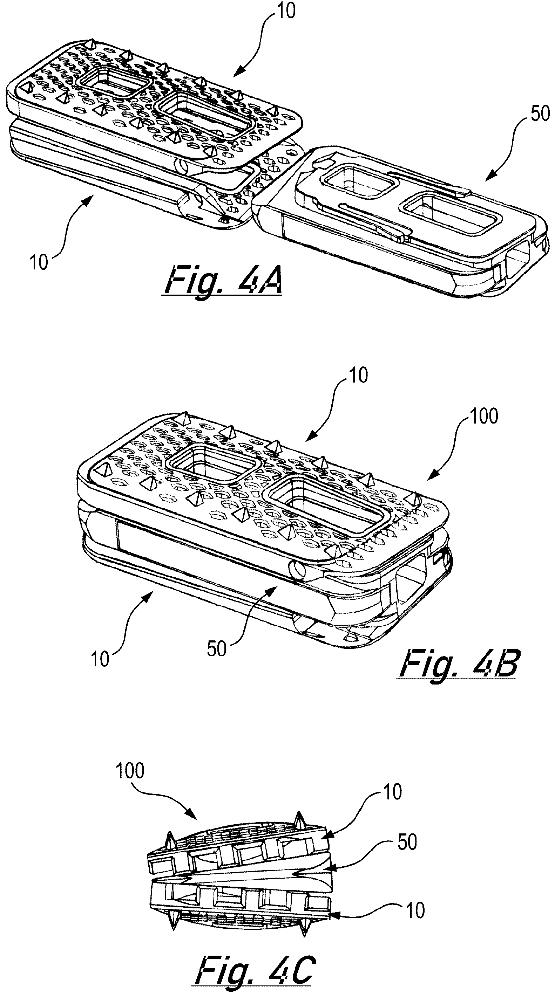

Installation and assembly of the LLIF device 100 will now be described with reference to Figures 4A to 4C. Figure 4A shows the core component 50 of Figures 3A to 3E before insertion between the superior and inferior components 10 of Figures 1A to 2F. Figure 4B shows the assembled LLIF device 100 after insertion of the core component 50 between the superior and inferior components 10. Figure 4C is a view of the second end of the assembled LLIF device 100.

The superior and inferior components 10 are placed against each other such that they are in registration and their first 34 and second 36 interdigitating projections interdigitate whereby the superior and inferior components are as shown in Figure 2A. The superior and inferior components 10 are mounted on an inserter instrument of known form and function. The superior and inferior components 10 are then introduced into the intervertebral space by the inserter instrument. The core component 50 is mounted on a core loader of known form and function and then introduced into the patient's body. The core loader is used to position the core component 50 such that the leading end 68 contacts the sloped portions 24 at the ends of the first and second superior rails 20, 22 on the superior and inferior components 10. Insertion of the core component 50 between the first ends 26 of the superior and inferior components 10 distracts the superior and inferior components. Further insertion of the core component 50 causes the upper 70 and lower 72 surfaces to bear against and slide over surfaces of the superior and inferior components 10 to orient and position the superior and inferior components relative to one another and to the core component to thereby align the first and second 56, 58 elongate protrusions with their respective channels 32 in the superior and inferior components. Further insertion of the core component 50 causes the first and second 56, 58 elongate protrusions to be received in and then slide along their respective channels 32 until the first interdigitating projection 34 is fully received in the projection recess 66 to arrest further insertion of the core component. At this stage of insertion, as shown in Figures 4B and 4C, the core component 50 is fully inserted between the superior and inferior components whereby the core component is in registration with the already registered superior and inferior components. Upon full insertion, the protrusions 62 on the end of the sprung arms 60 are received in their respective recesses 64 to present resistance to ejection of the core component 50 from between the superior and inferior components 10. The inserter instrument and the core loader are then disconnected from the superior and inferior components 10 and the core component 50, and the inserter instrument and the core loader are withdrawn from the patient's body.

## Claims

1. A modular lateral lumbar interbody fusion (LLIF) device (100) comprising:
a superior component (10) having a superior component top side (12) and a superior component bottom side (14), the superior component configured to be received in an intervertebral space between first and second vertebrae whereby the superior component top side abuts against the first vertebra;
an inferior component (10) having an inferior component top side (14) and an inferior component bottom side (12), the inferior component configured to be received in the intervertebral space between the first and second vertebrae whereby the inferior component bottom side abuts against the second vertebra, the superior component bottom side (14) and the inferior component top side (14) opposing each other when the superior and inferior components are received in the intervertebral space; and
a core component (50) configured for insertion between the superior and inferior components whereby a separation between the superior and inferior components and hence height of the LLIF device are determined,
wherein first and second superior rails (20, 22) protrude from the superior component bottom side (14), the first and second superior rails extending in the lateral direction, the first and second superior rails substantially parallel to each other and spaced apart from each other in a direction substantially orthogonal to the lateral direction,
wherein first and second inferior rails (20, 22) protrude from the inferior component top side (14), the first and second inferior rails extending in the lateral direction, the first and second inferior rails substantially parallel to each other and spaced apart from each other in a direction substantially orthogonal to the lateral direction, and
wherein the first and second superior rails and the first and second inferior rails are disposed on their respective components such that the first and second superior rails interdigitate with the first and second inferior rails when the superior and inferior components are in registration with and abut against each other before insertion of the core component between the superior and inferior components,
wherein a distal surface at a distal end of the first superior rail (20) bears against the inferior component top side (14) and a distal surface at a distal end of the first inferior rail (20) bears against the superior component bottom side (14) and the superior and inferior components are configured whereby the superior component top side and the inferior component bottom side are further apart from each other at first ends (26) than second opposite ends (28) of the superior and inferior components when the first and second superior rails interdigitate with the first and second inferior rails and before insertion of the core component between the superior and inferior components, and
the core component is configured to slidably inter-engage with each of the first and second superior rails and each of the first and second inferior rails during insertion of the core component between the superior and inferior components, the core component first received at the first ends (26) upon insertion of the core component

2. The modular lateral lumbar interbody fusion (LLIF) device according to claim 1, wherein the first and second superior rails (20, 22) are non-equidistantly spaced from a laterally extending plane which bisects the superior component (10), and the first and second inferior rails (20, 22) are non-equidistantly spaced from a laterally extending plane which bisects the inferior component (10).

3. The modular lateral lumbar interbody fusion (LLIF) device according to claim 2, wherein the first superior rail (20) is spaced apart from the laterally extending plane by a first rail distance further than the second superior rail (22) is spaced apart from the laterally extending plane, the first inferior rail (20) is spaced apart from the laterally extending plane by a second rail distance further than the second inferior rail (22) is spaced apart from the laterally extending plane, the first rail distance is no less than a width of each of the first and second inferior rails, and the second rail distance is no less than a width of each of the first and second superior rails.

4. The modular lateral lumbar interbody fusion (LLIF) device according to claim 2 or 3, wherein the first superior rail (20) extends width wise from a boundary on one side of the superior component (10), and the second superior rail (22) extends away from the laterally extending plane to a boundary distance short of a boundary on the other side of the superior component, the boundary distance of sufficient extent to receive the first inferior rail (20) and without the first inferior rail extending substantially beyond the boundary on the other side of the superior component.

5. The modular lateral lumbar interbody fusion (LLIF) device according to claim 4, wherein the first inferior rail (20) extends width wise from a boundary on one side of the inferior component (10), and the second inferior rail (22) extends away from the laterally extending plane to a boundary distance short of a boundary on the other side of the inferior component, the boundary distance of sufficient extent to receive the first superior rail (20) and without the first superior rail extending substantially beyond the boundary on the other side of the inferior component.

6. The modular lateral lumbar interbody fusion (LLIF) device according to any one of the preceding claims, wherein the superior and inferior components (10) are substantially the same at least in respect of disposition and size of their respective rails (20, 22).

7. The modular lateral lumbar interbody fusion (LLIF) device according to any one of the preceding claims, wherein each rail (20, 22) of the superior and inferior components (10) defines a channel (32) which extends along the rail and which faces towards a laterally extending plane of the respective component which bisects the component, each channel slidably receiving a respective elongate protrusion (56, 58) on the core component (50) during insertion of the core component between the superior and inferior components.

8. The modular lateral lumbar interbody fusion (LLIF) device according to claim 7, wherein each channel (32) is open at a first channel end and closed at a second channel end, the first channel ends of the first and second superior and inferior rails (20, 22) are at the first end (26) of the component, and the closed second channel ends of the first and second superior and inferior rails arrest movement of the core component relative to the superior and inferior components when the core component is in registration with the superior and inferior components.

9. The modular lateral lumbar interbody fusion (LLIF) device according to any one of the preceding claims, wherein a first plurality of interdigitating projections (34, 36) extends up from the superior component bottom side (14) between the first and second superior rails (20, 22) and a second plurality of interdigitating projections (34, 36) extends up from the inferior component top side (14) between the first and second inferior rails (20, 22), each of the first and second pluralities of interdigitating projections spaced apart in a direction orthogonal to the lateral direction, and each interdigitating projection extending in the lateral direction from the second end (28) of the respective superior or inferior component to no more than 20% across the respective superior or inferior component.

10. The modular lateral lumbar interbody fusion (LLIF) device according to claim 9, wherein first and second interdigitating projections (34, 36) extend up from each of the superior component bottom side (14) and the inferior component top side (14), the first and second interdigitating projections non-equidistantly spaced from a laterally extending plane which bisects the respective one of the superior and inferior components whereby the first and second interdigitating projections of the superior component interdigitate with the first and second interdigitating projections of the inferior component.

11. The modular lateral lumbar interbody fusion (LLIF) device according to claim 10, wherein the first interdigitating projection (34) is longer in the lateral direction than the second interdigitating projection (36), and a leading end of the first interdigitating projection is received, upon insertion of the core component between the superior and inferior components, in a correspondingly shaped recess (66) defined by the core component (50) to thereby present a barrier to further advancement of the core component between the superior and inferior components (10).

12. The modular lateral lumbar interbody fusion (LLIF) device according to any one of the preceding claims, wherein each of the first and second superior rails (20, 22) and the first and second inferior rails (20, 22) extends in the lateral direction from the second end (28) of the respective superior or inferior component (10) to at least 90% across the respective superior or inferior component, and a portion (24) of the end of each of the first and second superior rails and the first and second inferior rails towards the first end (26) of the respective superior or inferior component slopes down to the respective one of the superior component bottom side (14) and the inferior component top side (14) to thereby provide for ease of insertion of the core component between the superior and inferior components.

13. The modular lateral lumbar interbody fusion (LLIF) device according to any one of the preceding claims, wherein an inferior part (30) of the inferior component top side (14) against which the distal surface of the first superior rail (20) bears slopes downward in a direction from the first end (26) to the second end (28), and a superior part (30) of the superior component bottom side (14) against which the distal surface of the first inferior rail (20) bears slopes upward in the direction from the first end (26) to the second end (28) and when the rails of the superior and inferior components (10) interdigitate.

14. The modular lateral lumbar interbody fusion (LLIF) device according to claim 13, wherein there is progressive increase towards the second end (28) in the height of the second superior rail (22) relative to the superior part (30) and there is progressive increase towards the second end (28) in the height of the second inferior rail (22) relative to the inferior part (30).

15. The modular lateral lumbar interbody fusion (LLIF) device according to claim 13 or 14, wherein the superior part (30) increases in height relative to the superior component top side (12) and the inferior part (30) increases in height relative to the inferior component bottom side (12) in a direction from the second end (28) to the first end (26).

## Patentansprüche

1. Eine modulare laterale lumbale interkorporelle Fusionsvorrichtung (LLIF-Vorrichtung) (100), die Folgendes beinhaltet:
eine obere Komponente (10) mit einer oberen Komponentenoberseite (12) und einer oberen Komponentenunterseite (14), wobei die obere Komponente dazu ausgelegt ist, in einem Zwischenwirbelraum zwischen dem ersten und dem zweiten Wirbel aufgenommen zu werden, wodurch die obere Komponentenoberseite gegen den ersten Wirbel stößt;
eine untere Komponente (10) mit einer unteren Komponentenoberseite (14) und einer unteren Komponentenunterseite (12), wobei die untere Komponente dazu ausgelegt ist, in dem Zwischenwirbelraum zwischen dem ersten und dem zweiten Wirbel aufgenommen zu werden, wodurch die untere Komponentenunterseite gegen den zweiten Wirbel stößt, wobei die obere Komponentenunterseite (14) und die untere Komponentenoberseite (14) einander gegenüberliegen, wenn die obere und untere Komponente in dem Zwischenwirbelraum aufgenommen werden; und
eine Kernkomponente (50), die zum Einschub zwischen der oberen und unteren Komponente ausgelegt ist, wodurch eine Trennung zwischen der oberen und unteren Komponente und somit die Höhe der LLIF-Vorrichtung bestimmt wird,
wobei eine erste und zweite obere Schiene (20, 22) aus der oberen Komponentenunterseite (14) herausragen, wobei sich die erste und zweite obere Schiene in der lateralen Richtung erstrecken, wobei die erste und zweite obere Schiene im Wesentlichen parallel zueinander und voneinander beabstandet in einer im Wesentlichen zu der lateralen Richtung senkrechten Richtung verlaufen,
wobei die erste und zweite untere Schiene (20, 22) aus der unteren Komponentenoberseite (14) herausragen, wobei sich die erste und zweite untere Schiene in der lateralen Richtung erstrecken, wobei die erste und zweite untere Schiene im Wesentlichen parallel zueinander und voneinander beabstandet in einer im Wesentlichen zu der lateralen Richtung senkrechten Richtung verlaufen, und
wobei die erste und zweite obere Schiene und die erste und zweite untere Schiene so auf ihren jeweiligen Komponenten angeordnet sind, dass die erste und zweite obere Schiene mit der ersten und zweiten unteren Schiene ineinandergreifen, wenn die obere und untere Komponente sich decken und gegeneinander stoßen, bevor die Kernkomponente zwischen der oberen und unteren Komponente eingeschoben wird,
wobei eine distale Oberfläche an einem distalen Ende der ersten oberen Schiene (20) gegen die untere Komponentenoberseite (14) drückt und eine distale Oberfläche an einem distalen Ende der ersten unteren Schiene (20) gegen die obere Komponentenunterseite (14) drückt und die obere und untere Komponente solcherart ausgelegt sind, dass die obere Komponentenoberseite und die untere Komponentenunterseite an den ersten Enden (26) weiter voneinander entfernt sind als an den zweiten entgegengesetzten Enden (28) der oberen und unteren Komponente, wenn die erste und zweite obere Schiene mit der ersten und zweiten unteren Schiene ineinandergreifen und bevor die Kernkomponente zwischen der oberen und unteren Komponente eingeschoben wird, und
wobei die Kernkomponente dazu ausgelegt ist, während des Einschubs der Kernkomponente zwischen der oberen und unteren Komponente gleitend mit jeder von der ersten und zweiten oberen Schiene und jeder von der ersten und zweiten unteren Schiene einzugreifen, wobei die Kernkomponente nach dem Einschub der Kernkomponente zuerst an den ersten Enden (26) aufgenommen wird.

2. Modulare laterale lumbale interkorporelle Fusionsvorrichtung (LLIF-Vorrichtung) gemäß Anspruch 1, wobei die erste und zweite obere Schiene (20, 22) nicht im gleichen Abstand von einer sich lateral erstreckenden Ebene, die die obere Komponente (10) zweiteilt, beabstandet sind und die erste und zweite untere Schiene (20, 22) nicht im gleichen Abstand von einer sich lateral erstreckenden Ebene, die die untere Komponente (10) zweiteilt, beabstandet sind.

3. Modulare laterale lumbale interkorporelle Fusionsvorrichtung (LLIF-Vorrichtung) gemäß Anspruch 2, wobei die erste obere Schiene (20) von der sich lateral erstreckenden Ebene um einen ersten Schienenabstand beabstandet ist, der weiter ist als der, um den die zweite obere Schiene (22) von der sich lateral erstreckenden Ebene beabstandet ist, wobei die erste untere Schiene (20) von der sich lateral erstreckenden Ebene um einen zweiten Schienenabstand beabstandet ist, der weiter ist als der, um den die zweite untere Schiene (22) von der sich lateral erstreckenden Ebene beabstandet ist, wobei der erste Schienenabstand nicht weniger als eine Breite von jeder von der ersten und zweiten unteren Schiene beträgt und der zweite Schienenabstand nicht weniger als eine Breite von jeder von der ersten und zweiten oberen Schiene beträgt.

4. Modulare laterale lumbale interkorporelle Fusionsvorrichtung (LLIF-Vorrichtung) gemäß Anspruch 2 oder 3, wobei sich die erste obere Schiene (20) der Breite nach von einer Grenze auf einer Seite der oberen Komponente (10) erstreckt und sich die zweite obere Schiene (22) weg von der sich lateral erstreckenden Ebene bis zu einem Grenzabstand kurz vor einer Grenze auf der anderen Seite der oberen Komponente erstreckt, wobei der Grenzabstand ein ausreichendes Ausmaß hat, um die erste untere Schiene (20) aufzunehmen und ohne dass sich die erste untere Schiene im Wesentlichen über die Grenze auf der anderen Seite der oberen Komponente hinaus erstreckt.

5. Modulare laterale lumbale interkorporelle Fusionsvorrichtung (LLIF-Vorrichtung) gemäß Anspruch 4, wobei sich die erste untere Schiene (20) der Breite nach von einer Grenze auf einer Seite der unteren Komponente (10) erstreckt und sich die zweite untere Schiene (22) weg von der sich lateral erstreckenden Ebene bis zu einem Grenzabstand kurz vor einer Grenze auf der anderen Seite der unteren Komponente erstreckt, wobei der Grenzabstand ein ausreichendes Ausmaß hat, um die erste obere Schiene (20) aufzunehmen und ohne dass sich die erste obere Schiene im Wesentlichen über die Grenze auf der anderen Seite der unteren Komponente hinaus erstreckt.

6. Modulare laterale lumbale interkorporelle Fusionsvorrichtung (LLIF-Vorrichtung) gemäß einem der vorhergehenden Ansprüche, wobei die obere und untere Komponente (10) mindestens in Bezug auf Anordnung und Größe ihrer jeweiligen Schienen (20, 22) im Wesentlichen gleich sind.

7. Modulare laterale lumbale interkorporelle Fusionsvorrichtung (LLIF-Vorrichtung) gemäß einem der vorhergehenden Ansprüche, wobei jede Schiene (20, 22) der oberen und unteren Komponente (10) einen Kanal (32) definiert, der sich entlang der Schiene erstreckt und der einer sich lateral erstreckenden Ebene der jeweiligen Komponente, die die Komponente zweiteilt, zugewandt ist, wobei jeder Kanal während des Einschubs der Kernkomponente zwischen der oberen und unteren Komponente einen jeweiligen länglichen Vorsprung (56, 58) auf der Kernkomponente (50) gleitend aufnimmt.

8. Modulare laterale lumbale interkorporelle Fusionsvorrichtung (LLIF-Vorrichtung) gemäß Anspruch 7, wobei jeder Kanal (32) an einem ersten Kanalende offen und an einem zweiten Kanalende geschlossen ist, wobei die ersten Kanalenden der ersten und zweiten oberen und unteren Schienen (20, 22) an dem ersten Ende (26) der Komponente liegen und die geschlossenen zweiten Kanalenden der ersten und zweiten oberen und unteren Schienen die Bewegung der Kernkomponente relativ zu der oberen und unteren Komponente arretieren, wenn sich die Kernkomponente mit der oberen und unteren Komponente deckt.

9. Modulare laterale lumbale interkorporelle Fusionsvorrichtung (LLIF-Vorrichtung) gemäß einem der vorhergehenden Ansprüche, wobei sich eine erste Vielzahl von ineinandergreifenden Vorsprüngen (34, 36) von der oberen Komponentenunterseite (14) zwischen den ersten und zweiten oberen Schienen (20, 22) nach oben erstreckt und sich eine zweite Vielzahl von ineinandergreifenden Vorsprüngen (34, 36) von der unteren Komponentenoberseite (14) zwischen den ersten und zweiten unteren Schienen (20, 22) nach oben erstreckt, wobei jede von der ersten und zweiten Vielzahl von ineinandergreifenden Vorsprüngen in einer zu der lateralen Richtung senkrecht verlaufenden Richtung beabstandet ist und jeder ineinandergreifende Vorsprung sich in der lateralen Richtung von dem zweiten Ende (28) der jeweiligen oberen oder unteren Komponente bis zu nicht mehr als 20% über die jeweilige obere oder untere Komponente hinweg erstreckt.

10. Modulare laterale lumbale interkorporelle Fusionsvorrichtung (LLIF-Vorrichtung) gemäß Anspruch 9, wobei sich die ersten und zweiten ineinandergreifenden Vorsprünge (34, 36) von jeder von der oberen Komponentenunterseite (14) und der unteren Komponentenoberseite (14) nach oben erstrecken, wobei die ersten und zweiten ineinandergreifenden Vorsprünge nicht im gleichen Abstand von einer sich lateral erstreckenden Ebene, die die jeweilige eine von den oberen und unteren Komponenten zweiteilt, beabstandet sind, wodurch die ersten und zweiten ineinandergreifenden Vorsprünge der oberen Komponente mit den ersten und zweiten ineinandergreifenden Vorsprüngen der unteren Komponente ineinandergreifen.

11. Modulare laterale lumbale interkorporelle Fusionsvorrichtung (LLIF-Vorrichtung) gemäß Anspruch 10, wobei der erste ineinandergreifende Vorsprung (34) in der lateralen Richtung länger ist als der zweite ineinandergreifende Vorsprung (36) und ein vorderes Ende des ersten ineinandergreifenden Vorsprungs nach dem Einschub der Kernkomponente zwischen der oberen und unteren Komponente in einer entsprechend gestalteten Aussparung (66), die durch die Kernkomponente (50) definiert wird, aufgenommen wird, um somit eine Barriere gegen eine weitere Vorwärtsbewegung der Kernkomponente zwischen der oberen und unteren Komponente (10) darzustellen.

12. Modulare laterale lumbale interkorporelle Fusionsvorrichtung (LLIF-Vorrichtung) gemäß einem der vorhergehenden Ansprüche, wobei sich jede von der ersten und zweiten oberen Schiene (20, 22) und der ersten und zweiten unteren Schiene (20, 22) in der lateralen Richtung von dem zweiten Ende (28) der jeweiligen oberen oder unteren Komponente (10) bis zu mindestens 90% über die jeweilige obere oder untere Komponente hinweg erstreckt, und wobei ein Anteil (24) des Endes von jeder von der ersten und zweiten oberen Schiene und der ersten und zweiten unteren Schiene zu dem ersten Ende (26) der jeweiligen oberen oder unteren Komponente schräg nach unten zu der jeweiligen einen von der oberen Komponentenunterseite (14) und der unteren Komponentenoberseite (14) geneigt ist, um somit einen leichten Einschub der Kernkomponente zwischen der oberen und unteren Komponente bereitzustellen.

13. Modulare laterale lumbale interkorporelle Fusionsvorrichtung (LLIF-Vorrichtung) gemäß einem der vorhergehenden Ansprüche, wobei ein unterer Teil (30) der unteren Komponentenoberseite (14), gegen den die distale Oberfläche der ersten oberen Schiene (20) drückt, in einer Richtung von dem ersten Ende (26) zu dem zweiten Ende (28) schräg nach unten geneigt ist und wobei ein oberer Teil (30) der oberen Komponentenunterseite (14), gegen den die distale Oberfläche der ersten unteren Schiene (20) drückt, in der Richtung von dem ersten Ende (26) zu dem zweiten Ende (28) schräg nach oben geneigt ist, und wenn die Schienen der oberen und unteren Komponente (10) ineinandergreifen.

14. Modulare laterale lumbale interkorporelle Fusionsvorrichtung (LLIF-Vorrichtung) gemäß Anspruch 13, wobei es eine stetige Zunahme der Höhe der zweiten oberen Schiene (22) relativ zu dem oberen Teil (30) zu dem zweiten Ende (28) hin gibt und eine stetige Zunahme der Höhe der zweiten unteren Schiene (22) relativ zu dem unteren Teil (30) zu dem zweiten Ende (28) hin gibt.

15. Modulare laterale lumbale interkorporelle Fusionsvorrichtung (LLIF-Vorrichtung) gemäß Anspruch 13 oder 14, wobei die Höhe des oberen Teils (30) relativ zu der oberen Komponentenoberseite (12) zunimmt und die Höhe des unteren Teils (30) relativ zu der unteren Komponentenunterseite (12) in einer Richtung von dem zweiten Ende (28) zu dem ersten Ende (26) zunimmt.

## Revendications

1. Dispositif de spondylodèse lombaire par voie latérale (LLIF) modulaire (100) comprenant :
un composant supérieur (10) comportant un côté supérieur de composant supérieur (12) et un côté inférieur de composant supérieur (14), le composant supérieur étant conçu pour être reçu dans un espace intersomatique entre des première et seconde vertèbres de sorte que le côté supérieur de composant supérieur soit accolé à la première vertèbre ;
un composant inférieur (10) comportant un côté supérieur de composant inférieur (14) et un côté inférieur de composant inférieur (12), le composant inférieur étant conçu pour être reçu dans l'espace intersomatique entre les première et seconde vertèbres de sorte que le côté inférieur de composant inférieur soit accolé à la seconde vertèbre, le côté inférieur de composant supérieur (14) et le côté supérieur de composant inférieur (14) se faisant face lorsque les composants supérieur et inférieur sont reçus dans l'espace intersomatique ; et
un composant central (50) conçu pour être inséré entre les composants supérieur et inférieur de manière à définir un écartement entre les composants supérieur et inférieur et donc une hauteur du dispositif de LLIF,
dans lequel des premier et second rails supérieurs (20, 22) font saillie à partir du côté inférieur de composant supérieur (14), les premier et second rails supérieurs s'étendant dans la direction latérale, les premier et second rails supérieurs étant sensiblement parallèles entre eux et espacés l'un de l'autre dans une direction sensiblement orthogonale à la direction latérale,
dans lequel des premier et second rails inférieurs (20, 22) font saillie à partir du côté supérieur de composant inférieur (14), les premier et second rails inférieurs s'étendant dans la direction latérale, les premier et second rails inférieurs étant sensiblement parallèles entre eux et espacés l'un de l'autre dans une direction sensiblement orthogonale à la direction latérale, et
dans lequel les premier et second rails supérieurs et les premier et second rails inférieurs sont disposés sur leur composant respectif de telle sorte que les premier et second rails supérieurs s'engrènent avec les premier et second rails inférieurs lorsque les composants supérieur et inférieur sont mutuellement alignés et accolés avant l'insertion du composant central entre les composants supérieur et inférieur,
dans lequel une surface distale à une extrémité distale du premier rail supérieur (20) vient en appui contre le côté supérieur de composant inférieur (14) et une surface distale à une extrémité distale du premier rail inférieur (20) vient en appui contre le côté inférieur de composant supérieur (14) et les composants supérieur et inférieur sont conçus de sorte que le côté supérieur de composant supérieur et le côté inférieur de composant inférieur sont situés plus loin l'un de l'autre au niveau de premières extrémités (26) qu'ils ne le sont au niveau de secondes extrémités opposées (28) des composants supérieur et inférieur lorsque les premier et second rails supérieurs s'engrènent avec les premier et second rails inférieurs et avant l'insertion du composant central entre les composants supérieur et inférieur, et
le composant central est conçu pour entrer en prise mutuelle de manière coulissante avec chacun des premier et second rails supérieurs et chacun des premier et second rails inférieurs durant l'insertion du composant central entre les composants supérieur et inférieur, le composant central étant reçu en premier au niveau des premières extrémités (26) lors de l'insertion du composant central.

2. Dispositif de spondylodèse lombaire par voie latérale (LLIF) modulaire selon la revendication 1, dans lequel les premier et second rails supérieurs (20, 22) sont espacés de manière non équidistante d'un plan s'étendant latéralement qui coupe en deux le composant supérieur (10), et les premier et second rails inférieurs (20, 22) sont espacés de manière non équidistante d'un plan s'étendant latéralement qui coupe en deux le composant inférieur (10).

3. Dispositif de spondylodèse lombaire par voie latérale (LLIF) modulaire selon la revendication 2, dans lequel le premier rail supérieur (20) est séparé du plan s'étendant latéralement par une première distance de rail supérieure à la distance séparant le second rail supérieur (22) du plan s'étendant latéralement, le premier rail inférieur (20) est séparé du plan s'étendant latéralement par une seconde distance de rail supérieure à la distance séparant le second rail inférieur (22) du plan s'étendant latéralement, la première distance de rail n'est pas inférieure à une largeur de chacun des premier et second rails inférieurs, et la seconde distance de rail n'est pas inférieure à une largeur de chacun des premier et second rails supérieurs.

4. Dispositif de spondylodèse lombaire par voie latérale (LLIF) modulaire selon la revendication 2 ou 3, dans lequel le premier rail supérieur (20) s'étend dans la direction de la largeur à partir d'une limite sur un côté du composant supérieur (10), et le second rail supérieur (22) s'étend dans une direction s'éloignant du plan s'étendant latéralement jusqu'à une distance de limite, soit un point situé avant une limite sur l'autre côté du composant supérieur, la distance de limite étant suffisante pour recevoir le premier rail inférieur (20) sans que le premier rail inférieur ne s'étende sensiblement au-delà de la limite sur l'autre côté du composant supérieur.

5. Dispositif de spondylodèse lombaire par voie latérale (LLIF) modulaire selon la revendication 4, dans lequel le premier rail inférieur (20) s'étend dans la direction de la largeur à partir d'une limite sur un côté du composant inférieur (10), et le second rail inférieur (22) s'étend dans une direction s'éloignant du plan s'étendant latéralement jusqu'à une distance de limite, soit un point situé avant une limite sur l'autre côté du composant inférieur, la distance de limite étant suffisante pour recevoir le premier rail supérieur (20) sans que le premier rail supérieur ne s'étende sensiblement au-delà de la limite sur l'autre côté du composant inférieur.

6. Dispositif de spondylodèse lombaire par voie latérale (LLIF) modulaire selon l'une quelconque des revendications précédentes, dans lequel les composants supérieur et inférieur (10) sont sensiblement identiques au moins en ce qui concerne la disposition et la taille de leurs rails respectifs (20, 22).

7. Dispositif de spondylodèse lombaire par voie latérale (LLIF) modulaire selon l'une quelconque des revendications précédentes, dans lequel chaque rail (20, 22) des composants supérieur et inférieur (10) définit un canal (32) qui s'étend le long du rail et est orienté vers un plan s'étendant latéralement du composant respectif qui coupe en deux le composant, chaque canal recevant de manière coulissante une saillie allongée respective (56, 58) sur le composant central (50) durant l'insertion du composant central entre les composants supérieur et inférieur.

8. Dispositif de spondylodèse lombaire par voie latérale (LLIF) modulaire selon la revendication 7, dans lequel chaque canal (32) est ouvert au niveau d'une première extrémité de canal et fermé au niveau d'une seconde extrémité de canal, les premières extrémités de canal des premiers et seconds rails supérieurs et inférieurs (20, 22) se trouvent au niveau de la première extrémité (26) du composant, et les secondes extrémités de canal fermées des premiers et seconds rails supérieurs et inférieurs arrêtent le déplacement du composant central relativement aux composants supérieur et inférieur lorsque le composant central est aligné avec les composants supérieur et inférieur.

9. Dispositif de spondylodèse lombaire par voie latérale (LLIF) modulaire selon l'une quelconque des revendications précédentes, dans lequel une première pluralité de proéminences d'engrènement (34, 36) fait saillie à partir du côté inférieur du composant supérieur (14) entre les premier et second rails supérieurs (20, 22) et une seconde pluralité de proéminences d'engrènement (34, 36) fait saillie à partir du côté supérieur du composant inférieur (14) entre les premier et second rails inférieurs (20, 22), chacune des première et seconde pluralités de proéminences d'engrènement étant espacée dans une direction orthogonale à la direction latérale, et chaque proéminence d'engrènement s'étendant dans la direction latérale à partir de la seconde extrémité (28) du composant supérieur ou inférieur respectif sur au plus 20 % du composant supérieur ou inférieur respectif.

10. Dispositif de spondylodèse lombaire par voie latérale (LLIF) modulaire selon la revendication 9, dans lequel des première et seconde proéminences d'engrènement (34, 36) font saillie à partir de chacun du côté inférieur de composant supérieur (14) et du côté supérieur de composant inférieur (14), les première et seconde proéminences d'engrènement étant espacées de manière non équidistante d'un plan s'étendant latéralement qui coupe en deux le composant respectif parmi les composants supérieur et inférieur, de sorte que les première et seconde proéminences d'engrènement du composant supérieur s'engrènent avec les première et seconde proéminences d'engrènement du composant inférieur.

11. Dispositif de spondylodèse lombaire par voie latérale (LLIF) modulaire selon la revendication 10, dans lequel la première proéminence d'engrènement (34) est plus longue dans la direction latérale que la seconde proéminence d'engrènement (36), et une extrémité antérieure de la première proéminence d'engrènement est reçue, lors de l'insertion du composant central entre les composants supérieur et inférieur, dans un renfoncement de forme correspondante (66) défini par le composant central (50) pour faire ainsi obstacle à un déplacement plus avant du composant central entre les composants supérieur et inférieur (10).

12. Dispositif de spondylodèse lombaire par voie latérale (LLIF) modulaire selon l'une quelconque des revendications précédentes, dans lequel chacun des premier et second rails supérieurs (20, 22) et des premier et second rails inférieurs (20, 22) s'étend dans la direction latérale à partir de la seconde extrémité (28) du composant supérieur ou inférieur respectif (10) sur au moins 90 % du composant supérieur ou inférieur respectif, et une partie (24) de l'extrémité de chacun des premier et second rails supérieurs et des premier et second rails inférieurs vers la première extrémité (26) du composant supérieur ou inférieur respectif est biseautée en direction du côté respectif parmi le côté inférieur de composant supérieur (14) et le côté supérieur de composant inférieur (14) de façon à faciliter l'insertion du composant central entre les composants supérieur et inférieur.

13. Dispositif de spondylodèse lombaire par voie latérale (LLIF) modulaire selon l'une quelconque des revendications précédentes, dans lequel une partie inférieure (30) du côté supérieur de composant inférieur (14) contre laquelle la surface distale du premier rail supérieur (20) vient en appui est inclinée vers le bas dans une direction allant de la première extrémité (26) à la seconde extrémité (28), et une partie supérieure (30) du côté inférieur de composant supérieur (14) contre laquelle la surface distale du premier rail inférieur (20) vient en appui est inclinée vers le haut dans la direction allant de la première extrémité (26) à la seconde extrémité (28) et lorsque les rails des composants supérieur et inférieur (10) sont engrenés.

14. Dispositif de spondylodèse lombaire par voie latérale (LLIF) modulaire selon la revendication 13, dans lequel la hauteur du second rail supérieur (22) relativement à la partie supérieure (30) augmente progressivement en direction de la seconde extrémité (28) et la hauteur du second rail inférieur (22) relativement à la partie inférieure (30) augmente progressivement en direction de la seconde extrémité (28).

15. Dispositif de spondylodèse lombaire par voie latérale (LLIF) modulaire selon la revendication 13 ou 14, dans lequel la hauteur de la partie supérieure (30) relativement au côté supérieur de composant supérieur (12) augmente et la hauteur de la partie inférieure (30) relativement au côté inférieur de composant inférieur (12) augmente dans une direction allant de la seconde extrémité (28) à la première extrémité (26).
